# EUROPEAN PATENT SPECIFICATION

(11) **EP 1 084 232 B1**
(45) Date of publication and mention of the grant of the patent: **28.04.2004**
(21) Application number: 99923531.0
(22) Date of filing: 05.05.1999
(51) Int. Cl.: C12N 5/10

(54) **GENETICALLY-MODIFIED FIBROBLASTS AND THE USE THEREOF**
GENETISCH VERÄNDERTE FIBROBLASTEN UND IHRE VERWENDUNG
FIBROBLASTES GENETIQUEMENT MODIFIES ET UTILISATION

(30) Priority: 08.05.1998 IT MI981004
(43) Date of publication of application: 21.03.2001
(73) Proprietor: Molecular Medicine S.p.A., 20132 Milano (IT)
(72) Inventor: MAVILIO, Fulvio, I-20132 Milano (IT)
(74) Representative: Minoja, Fabrizio, Dr.
(86) International application number: PCT/EP1999/003075
(87) International publication number: WO 1999/058646

(56) References cited:
- WO-A-95/12979
- WO-A-96/09373
- WO-A-98/17784
- US-A- 5 219 740
- WEINTRAUB H ET AL: "ACTIVATION OF MUSCLE-SPECIFIC GENES IN PIGMENT, NERVE, FAT, LIVER, AND FIBROBLAST CELL LINES BY FORCED EXPRESSION OF MYOD" PROCEEDINGS OF THE NATIONAL ACADEMY OF SCIENCES OF USA, vol. 86, 1 July 1989 (1989-07-01), pages 5434-5438, XP000672860 ISSN: 0027-8424
- LATTANZI L, ET AL.: "High Efficiency Myogenic Conversion of Human Fibroblasts by Adenoviral Vector-Mediated MyoD gene transfer" JOURNAL OF CLINICAL INVESTIGATION, vol. 101, no. 10, 15 May 1998 (1998-05-15), pages 2119-2128, XP002112711
- HUARD C, ET AL.: "Transplantation of Dermal Fibroblasts Expressing MyoD1 in Mouse Muscles " BIOCHEMICAL AND BIOPHYSICAL RESEARCH COMMUNICATIONS, vol. 248, no. 3, 30 July 1998 (1998-07-30), pages 648-654, XP002112712

## Description

The present invention relates to an ex-vivo method for the myogenic conversion of genetically-modified fibroblasts, for use in the correction of congenital defects of the muscular system, such as primary monogenic myopathies, or in the production of therapeutical proteins. Furthermore, the invention relates to the use of genetically-modified fibroblasts for the preparation of compositions for cell implants for the treatment of muscle pathologies with a genetic aetiology, or for the in vivo secretion of recombinant proteins of therapeutical importance. In particular, the invention is valuable for the treatment of muscular dystrophies, a heterogeneous group of severe degenerative diseases of the muscle caused by mutations in the genes encoding for the membrane-associated protein dystrophin or for other members of the dystrophin-associated protein complex connecting the muscle fiber cytoskeleton with the extracellular matrix.

Until now, the attempts to correct muscular dystrophy with gene therapy based on the implantation of muscles or other tissues stem cells, have been thwarted by enormous difficulties in the isolation of a suitable number of myogenic cells to be genetically-modified or to the poor yield of phenotypic conversion. By way of example, the difficulty in obtaining a reasonable number of satellite cells to be genetically-modified is a limit to gene therapy of patients with Duchenne muscular dystrophy or with Becker muscular dystrophy (Salvatori, G., et al., 1993; Webster, C. et al., 1990). Further problems related to the implantation of genetically-modified cells in dystrophic patients are the immunogenicity of viral vectors, the difficulty in developing an effective administration route and the poor survival of injected cells. Furthermore, the expression of dystrophin itself has been reported to induce cellular and/or humoral immune responses in dystrophin-lacking patients (New England J. of Medicine 333, 732-733, 1995).

It has been known for some time that forced expression of MyoD, and related members of the myogenic family of basic helix-loop-helix transcription factors, would activate myogenesis in non-muscle cells (Davis, R. L., et al., 1987; Cossu, G., et al., 1996; Cossu, G., 1997). The Applicant itself has recently reported that fibroblasts undergo myogenic differentiation when co-cultured with myogenic lines (C2C12) or primary cells, but not with other cell types (Breton, M. et al., 1995; Gibson, A.J., et al., 1995; Salvatori, G. et al., 1995). WO 95/12979 discloses a general method for inducing cell differentiation to a novel phenotype, for example muscular phenotype, by inserting the genic sequences of factors such as MyoD, myogenin, Myf-5 and MRF4 into the cells. The same document neither considers any possibility to convert genetically-modified cells to the myogenic phenotype in order to make them suitable for gene therapy, nor envisages the possibility to carry out an "ex vivo" myogenic cqnversion.

GB 2293604 discloses the use of fibroblasts in the treatment of muscular pathologies, wherein fibroblasts themselves can be transformed with a therapeutically active gene, for example the dystrophin gene, whereas the possibility to induce differentiation by means of myogenic factors has not been considered.

In light of what stated above, it appears remarkably advantageous to rely on a method which allows a high number of cells to be converted to the myogenic phenotype, after being genetically-modified ex-vivo, in order to correct a genetic defect, such as muscular dystrophy, or any other dysfunction related, for instance, to insufficient or incorrect production of a protein or a plasma factor or other secreted or circulating proteins, as in the case of diabetes, hemophilia, pituitary dwarfism.

It has now been found that it is possible to convert genetically-modified fibroblasts to the muscular phenotype, with a high-efficiency process, so as to effectively produce therapeutical proteins or proteins able to correct the genetic defects, particularly muscular dystrophy.

According to a first aspect, the invention provides a high-efficiency method for the preparation of genetically-modified fibroblasts expressing muscle lineage commitment genes, which comprises:
a) ex-vivo transduction of fibroblasts with a therapeutic gene or a gene capable of correcting a genetic defect;
b) transient expression of the muscle lineage commitment gene in fibroblasts transduced as at point (a), through transformation of the cells with a high efficiency DNA transfer method, preferably a viral vector, more preferably selected from baculovirus, adeno-associated viruses and, most preferably, adenovirus, or with other high efficiency DNA transfer means, such as liposomes, nucleoprotein complexes, polyethylenimines, wherein the gene of the muscle lineage commitment is under the control of a strong promoter, preferably a viral promoter.

"Muscle lineage commitment gene" means any gene capable of converting fibroblasts to the myogenic phenotype, particularly MyF-5, MRF4, myogenin and, preferably, MyoD genes.

Therapeutical genes which can be transduced into fibroblasts before the myogenic conversion are, for example, genes involved the various types of muscle dystrophies, such as sarcoglycan, emerin and, preferably, dystrophin genes, or genes coding for circulating plasma proteins, such as coagulation factors or insulin, for hormones, such as growth hormone, or other genes, such as hypoxanthine-guanine phosphoribosyl transferase, adenosine deaminase, purine nucleoside phosphorylase, glucocerebrosidase, low density lipoprotein receptor, phenylalanine hydroxylase, arginine succinate synthetase and aryl-sulfatase.

A second aspect of the invention relates to genetically-modified fibroblasts, transiently expressing a gene of the muscle lineage commitment, preferably MyoD, obtained by the method of the invention.

The conversion of fibroblasts to the myogenic phenotype by transient expression of a muscle lineage commitment gene, proved to be surprisingly more effective than other alternative techniques. In fact, co-culture of fibroblasts with satellite cells, treatment with dexamethasone and transfection with calcium phospnate, lipofectamine, or electroporation of the same plasmid expressing MyoD, induced myogenic conversion in a percentage ranging from 1% to 14%, in comparison with values higher than 70% in case of infection with a recombinant adenoviral vector. Furthermore, the efficiency of conversion was found to increase linearly for m.o.i. ("multiplicity of infection") values from 500 to 2000, whereas cytotoxicity at maximum m.o.i. values, remained within acceptable limits. Similar results were obtained with human or murine fibroblasts, whereas as far as tissutal fibroblasts are concerned, dermal fibroblasts gave better results. One main advantage of the method of the invention is that transient expression of the exogenous muscle lineage-commitment gene activates the corresponding endogenous gene, therefore irreversibly committing cells to myogenesis, thus rendering constitutive expression of the transgene unnecessary. The actual myogenic conversion of fibroblasts transiently expressing exogenous MyoD has been confirmed by immuno-histochemistry, electron microscopy and gene expression analysis of different muscle-specific markers, such as myosin light and heavy chains, acetylcholine receptor α-subunit, M creatine kinase, myogenin and MyoD.

The high efficiency of the method allows the preparation of genetically manipulated fibroblasts in which a therapeutic gene, or a gene correcting the genetic defect in the muscle has been inserted, which may be stably converted to the myogenic phenotype. In fact, conversion to the muscle phenotype prevents cell replication and provides stable implants, based on the use of muscle fibers.

Furthermore, the efficiency of the method as well as the stability of the resulting product allow to successfully carry out gene-therapy, in which genetically-modified fibroblasts expressing a gene of the muscle lineage commitment, after appropriate ex-vivo treatment, are injected into muscle tissue where they are capable of regenerating the muscle fibers and express the correct gene.

A number of evidences proved that fibroblasts treated ex-vivo as herein disclosed are able to regenerate muscle fibers in mice, indistinguishable from those originating from primary myogenic (satellite) cells, with a long-lasting effect. Moreover, after treatment, only antibody mediated immune response against proteins of adenoviral infected fibroblasts has been detected. No cell-mediated immune response has been observed at the site of injection of the modified fibroblasts. This is a significant advantage in gene therapy, as one of the major problems associated to the in vivo use of adenoviral vectors is the induction of significant, T-cell-mediated immune responses against both the viral and transgenic proteins.

According to a further aspect, the invention relates to the use of fibroblasts obtained by the above described method, for "ex vivo" gene therapy or for the preparation of compositions for stable cell implants based on muscle fibers.

A typical application of the ex-vivo gene therapy comprises, for example, isolating dermal fibroblasts from a patient suffering from a genetic disease which alters the muscle structure or functionality, such as dystrophy, expanding the cultured cells, transducing the cells ex-vivo with a retroviral vector containing the correct gene, and inducing myogenesis by infection with an adenoviral vector, or with another vector as mentioned above, containing the gene of the muscle lineage commitment, and re-implanting modified cells into the muscle tissue.

Another typical approach for the preparation of cell implants based on muscle fibers comprises isolating dermal fibroblasts from a patient suffering from a disease characterised by, for example, lack of a plasma protein (such as diabetes, pituitary dwarfism, hemophilia), expanding the cultured cells, transducing the cells ex-vivo with a retroviral vector containing the therapeutic gene, and inducing myogenesis by infection with an adenoviral vector, or with another vector as mentioned above, containing the muscle lineage commitment gene, encapsulating the modified cells in the most suitable implantation matrix and implanting the preparation in the preferred body site. In this specific application, induction of myogenesis and subsequent differentiation into muscle tissue is not aimed at the restoration of muscle functionality, but to the stability of the implant by arrest of cell division.

As further applications of the present invention, its use in zootechnic field can be envisaged, to induce a specific characteristic in animals, for example an increase in weight, by production of a hormone or any other substance by the cells treated ex vivo with the herein disclosed method and then re-implanted into the animal; or the creation or use of animal models of human pathologies for the study of novel therapeutical procedures to be carried out with the method of the invention.

The following examples illustrate the invention in greater details.

### Example 1

### Preparation of genetically-modified fibroblasts expressing MyoD.

Fetal fibroblasts were isolated from skin and skeletal muscle of C3H mouse embryos (15-17 days) or legally aborted human fetuses (8-12 weeks) as described in Salvatori et al., *Human Gene Ther*., 1993, 4:713-723, or human dermal or muscle fibroblasts were obtained from tissues of patients undergoing post-traumatic surgery. In this case tissues were finely minced with scissors, digested with 2 mg/ml dispase, 0.1 mg/ml collagenase in phosphate buffered saline (PBS) for 45' at 37°C, washed in RPMI medium, and pipetted to obtain a single-cell suspension.

Murine bone marrow fibroblasts transgenic for the lacZ gene with nuclear localization, were obtained by resuspending and plating cells flushed from the long bones of 8-10-wk-old MLC3F/nlacZ mice. Human bone marrow fibroblasts were obtained from healthy donors after removal of non-adherent cells. All cells were grown in RPMI supplemented with 15% Fetal calf serum (FCS), 1% gentamycin and 0.3 mM β-mercaptoethanol (growth medium). Human bone marrow fibroblasts were supplemented with 2 ng/ml bFGF.

Myogenic differentiation was induced by shifting the cells to RPMI supplemented with 2%.horse serum (differentiation medium). Fibroblasts were purified by sub-culture (at least two rounds) in growth medium. Removal of myogenic cells was routinely verified by immunocytochemical staining of a cell aliquot sub-cultured for 5 days in differentiation medium.

For transduction, fibroblasts (from third to tenth passage) were infected with a replication-defective retroviral vector (LBSN) expressing a cytoplasmic β-galactosidase gene under the LTR promoter as described (Salvatori, G., et al., 1993).

For the subsequent step (transient expression of MyoD), cells were treated with the Ad5-derived, E1A-deleted adenoviral vector expressing the full-length murine MyoD c-DNA under the transcriptional control of Rous Sarcoma Virus (RSV) LTR (Murry, C. E., et al., 1996). In some control experiments, cells were transfected by standard precipitation techniques with calcium-phosphate or lipofectamine (Dotap) with 10 µg of the PMC11 plasmid, containing the MyoD cDNA under the control of the CMV promoter.

Alternatively, cells were electroporated with 6 µg of the same plasmid in growth medium at 120 V, 960 mF. After transduction, cells were grown for 24 hrs in growth medium and then either shifted to differentiation medium for 3-4 days or injected in vivo.

In some experiments, cells were pre-labelled with 0.5 mCi/ml of [14C] thymidine (Amersham) for 24 h and then exposed to the adenoviral vector expressing MyoD and survival was measured by counting residual cpm incorporated.

### Example 2

### Implantation of MyoD-converted fibroblasts into mouse muscles.

1 or 2 millions of transduced (or transgenic) human or murine fibroblasts, converted by transient expression of MyoD, were trypsinized, resuspended in 20-50 µl of PBS and injected into a single site of the regenerating tibialis anterior muscle of either syngeneic (C3H) or immunodeficient (scid/bg) mice which had received a 30-µl injection of 10-5 M cardiotoxin (Latoxan) 48 hrs earlier.

For some experiments, myogenically converted mouse fibroblasts were labelled with DiI dye 1 hr before injection in vivo (a 0.5% solution of DiI in absolute ethanol was diluted just before use in 0.3 M sucrose to a final concentration of 0.05%).

Mice were sacrificed after various periods of time, and muscles were cryo-sectioned and stained for β-galactosidase activity or processed for immuno-hystochemistry. At the time of sacrifice, serum was collected from C3H mice and reacted with adeno-infected or control cells at various dilutions, followed by a fluorescein-conjugated anti-mouse IgG secondary antibody.

### Example 3

### Immunocytochemistry, electron microscopy and RNA analysis techniques.

Immunofluorescence analysis was carried out as described (Cusella - De Angelis, M.G., et al., 1994) using the following antibodies: MF20, a monoclonal antibody which recognises all sarcomeric myosins (Bader, D., et al., 1982); a rabbit antiserum against sarcomeric proteins (Tajbakhrsh, S., et al., 1994); an anti-MyoD polyclonal antibody (Hasty, P., et al., 1993); BD5, a monoclonal antibody which recognises slow myosin heavy chains (Yasin, R., et al., 1977); a rabbit polyclonal antibody against human fetal myosin (Edom, F., et al., 1994); an anti-bromo-deoxyuridine (BrdU) monoclonal antibody; anti-leu, anti-CD4 and anti-Mac3 rat anti-mouse leukocyte antigen antibodies. Briefly, cells cultures or cryostat sections were fixed with 4% paraformaldehyde for 10 min at 4°C, washed 3X in PBS, and incubated at 4°C with the primary antibodies in PBS + 1% bovine serum albumin (BSA). BrdU labelled cell-cultures were treated for 10 min at rt with 2M HCl and washed 3X with PBS before incubation with the anti-BrdU antibody.

After the first incubation, cells or sections were washed 3X with PBS + 1% BSA and incubated for 1 hr at rt with rhodamine-conjugated goat anti-mouse Ig or with a fluorescein-conjugated goat anti rabbit Ig (1:100 dilution).

Cultures or sections were then washed, mounted in 75% glycerol/PBS (pH 7.5), and observed under a Zeiss Axiophot epifluorescence microscope.

For electron microscopy cells were washed in PBS, fixed in 2% glutaraldehyde in 0.1 M Millonig's buffer (pH 7.4) for 1 hr at 4°C, post-fixed for 1 hr in 1% buffered osmium tetroxide and dehydrated in graded alcohol. Cells were treated with propylene oxide, embedded in Epon 812 and cut into ultrathin sections. The ultrathin sections were stained with uranyl acetate and lead citrate, and examined with a Zeiss 109 electron microscope.

For RNA analysis, RNA was extracted by the method of Chomczynski et al. (Chomczynski, P., et al., 1987), run in 15 µg aliquots on 1% agarose/formaldehyde gels, and transferred by capillary Northern blotting to nylon membranes (Amersham Hybond-N).

Filters were cross-linked for 2 hrs at 80 °C under vacuum, and hybridized to [32P]-labelled probes for MyoD and myogenin (Bober, E., et al., 1991), MLC1F and MCK (Lyons, G. E., et al., 1991), Ach-receptor α-subunit (25) under standard conditions (26).

### Example 4

### Analysis of in vitro converted fibroblasts expressing the myogenic phenotype.

In order to compare the adeno-MyoD-induced phenotype with that of primary myogenic cells, a number of muscle-specific markers were analysed by immuno-cytochemistry, electron microscopy and gene expression analysis, in human and murine converted dermal fibroblasts, and in differentiating satellite cells of comparable age.

All differentiated myotubes derived from fusion of converted murine newborn dermal fibroblasts express fast embryonic myosin heavy chains, while a fraction of them also express slow myosin heavy chains, while a fraction of them also express slow myosin heavy chains, as observed in myotubes derived from satellite cells.

Similarly, myotubes derived from human fetal dermal fibroblasts show a well organized initial sarcomerogenesis, with aligned sarcomeres and patterned Z lines, as reported for myotubes derived from primary myogenic cells, which never complete sarcomerogenesis in vitro.

In order to verify whether the introduction of MyoD in primary fibroblasts would also activate transcription of genes responsible for membrane and metabolic muscle-specific functions, the Applicant measured by Northern blotting the expression of RNAs coding muscle-specific proteins such as the acetylcholine (ACh) receptor α-subunit, M creatine kinase (MCK), as well as MyoD, myogenin, and myosin light chain 1 fast (MLC1F) chosen as positive controls.

No detectable level of any of these messages was detected in unconverted fibroblasts; on the other hand, these mRNAs were expressed at comparable levels in myotubes derived from converted fibroblasts and in myotubes derived from satellite cells of corresponding age.

Thus, under all the parameters analysed in vitro, myotubes originating from converted fibroblasts cannot be distinguished from those derived by primary myogenic cells.

Afterwards, the Applicant investigated whether fibroblasts exposed to the MyoD adenoviral vector would still maintain the capacity to divide, since MyoD overexpression has an anti-proliferative effect. Murine and human fetal skin fibroblasts were infected with the MyoD adenoviral vector for 3 hrs at an m.o.i. of 2,000 in serum-free medium, cultured for 12 hrs in growth medium containing 10 mM BrdU, fixed at 12, 24, 48 and 72 hrs after infection, and double-stained with anti-MyoD and anti-BrdU antibodies.

24 hrs after exposure to the adenoviral vector, only a few cells expressing MyoD had incorporated BrdU, while no double-positive cells were detected at 48 and 72 hrs. Expression of MyoD therefore blocks cell division in converted fibroblasts.

### Example 5

### Myogenic conversion efficiency of human and murine fibroblasts of different origins

Fibroblasts were isolated, expanded, infected with the MyoD adenoviral vector for 3 hrs at an m.o.i. of 2,000, cultured for a further 24 hrs and then induced to differentiation as described. Table 1 shows the conversion % values.

The conversion efficiency was measured as percentage of cells which expressed sarcomeric myosin heavy chains, as shown by staining with anti-myosin antibody. (MF20). Values shown are the average of two separate experiments, each performed in triplicate.

**Table 1**

| | Murine | | Human | |
|---|---|---|---|---|
| | Fetal | Adult | Fetal | Adult |
| Derma | 70 | 56 | 65 | 42 |
| Muscle | 43 | 44 | 40 | 32 |
| Bone marrow | ND | 6 | ND | 6 |
| ND: not done. | | | | |

### Example 6

### Survival of MyoD-converted fibroblasts and satellite cells (from MLC3F/LacZ mice) after injection into regenerating Tibialis Anterior muscle (TA) of SCID/bg mice.

20 µl containing 1x10⁶ cells were injected into a single site of regenerating TA of SCID/bg mice.

At the indicated times, mice were sacrificed and successive 15 µm cryostat sections were prepared from the TA muscle. 1 every 5 slides was X-gal stained. Nuclei were counter-stained with Hoechst. The X-gal stained sections, were scored for the number of β-gal⁺ nuclei and the obtained values were multiplied x 5 (total No. of nuclei). In addition β-gal⁺/total nuclei in 200 µ² areas was counted. Results are reported in Table 2. Note that the increase in the β-gal⁺/total nuclei ratio is due to the decrease in monunucleated cells which progressively occurs during muscle regeneration (fusion).

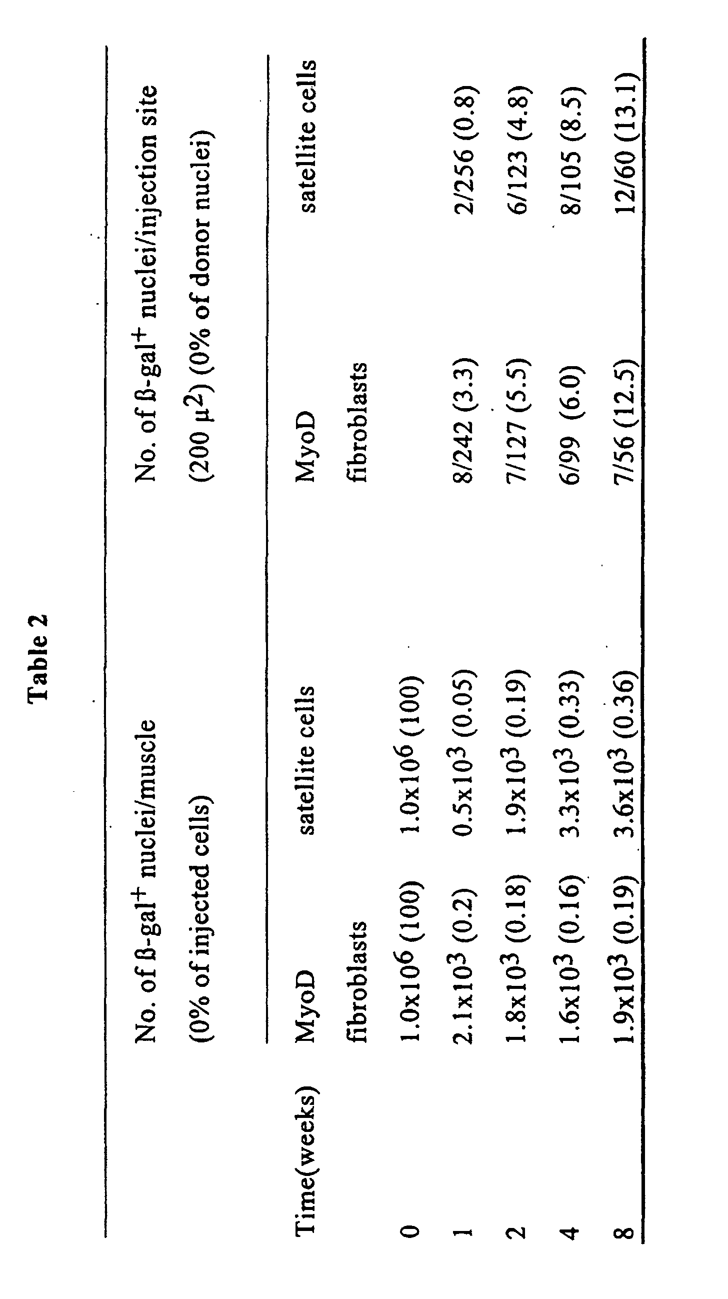

### Example 7

### Muscle regeneration by genetically-modified fibroblasts expressing MyoD

Fibroblasts isolated from human fetal skin were expanded and transduced in vitro with a high-titer stock of the retroviral vector LBSN, carrying a lacZ gene encoding a cytoplasmic form of β-galactosidase (Salvatori, G., et al., 1993).

Transduction efficiency, estimated by the number of β-gal-staining cells in culture, ranged between 40 and 70%, making further selection of the transduced cells (e.g., G418 resistance) unnecessary. Transduced fibroblasts were exposed to the adeno-MyoD vector at an m.o.i. of 2,000, and then injected into the regenerating TA muscles of scid/bg mice (106 cells/muscle in a single injection).

The efficiency of myogenic conversion of transduced fibroblasts was checked by allowing part of the cell culture to differentiate in vitro. On average, about 70% of the fibroblasts underwent myogenic conversion in these conditions, and most myotubes (> 90 %) scored positive for cytoplasmic β-gal expression. After 1, 2 and 4 weeks, mice were sacrificed and the TA serially sectioned and tibialis anterior muscles were cut and stained for β-gal activity.

Two weeks after injection, β-gal+ fibers were observed in 7 out of 8 injected muscles. Higher magnification clearly revealed fibers accumulating the reporter gene product at variable levels, suggesting a variability in the proportion of injected/host cells in the β-gal+ fibers.

The presence and contribution of human cells in the newly formed fibers was confirmed by immunocytochemical analysis using an antibody which recognizes human but not mouse fetal myosin heavy chains, which showed patches of intensive expression of human muscle-specific proteins in the regenerating areas.

In general, the average number of β-gal+ fibers per muscle obtained by injecting human converted fibroblasts was lower than that observed after injection of murine fibroblasts, although comparable to the number of positive cells observed in experiments in which lacZ-transduced human satellite cells were used. This indicates that human cells are, as expected, less efficient than murine ones in colonizing a mouse muscle.

However, and more importantly, human converted fibroblasts perform like primary myogenic cells in vivo, in terms of both number and size of newly formed fibers.

In order to better quantify the survival of human myogenically converted fibroblasts in mouse muscle, the Applicant also performed dot-blot Southern analysis with an Alu probe, as previously described (Salvatori, G., et al., 1993). To this purpose DNA was extracted from TA muscles of scid/bg mice two weeks after the injection of MyoD-converted human fibroblasts (following the same experimental protocol described above). As a positive control, the same number of human satellite cells was injected in similarly treated contra-lateral TA muscles.

The results of the dot-blot indicated that approximately 0.1% of the DNA extracted from TA injected with converted fibroblasts was of human origin; on the other hand this value raised to 0.3% in the case of the sample injected with human satellite cells.

Because histochemical analysis had revealed that virtually all the β-galactosidase staining was inside muscle fibers, we can conclude that the observed values faithfully reflect the percentage of human nuclei incorporated into regenerated muscle fibers.

### Example 8

### Immune response against cells exposed ex vivo to the adenoviral vector.

One of the major problems associated to the in vivo use of adenoviral vectors in immunocompetent recipients is the induction of a significant, T-cell-mediated immune response against both the viral proteins and the product of the transgene(s). In the model described herein, no viral particle is injected directly in vivo. However, injected cells had been exposed shortly before administration to the adenoviral vector in vitro.

Replication-defective adenoviral DNA is expected to be rapidly diluted, and eventually lost, in cells in active cell division. Expression of the MyoD transgene, however, blocks almost immediately cell division in the converted fibroblasts, thus preventing the possibility of diluting the adenoviral vector by continuous culture.

To test whether an immune response could be raised by cells expressing a defective adenoviral genome in vivo, the Applicant injected 2 x 10⁶ C3H murine skin fibroblasts converted to myogenesis by exposure to the adeno-MyoD vector in the regenerating muscle of singeneic mice.

Cells were labelled with DiI before injection and not with a lacZ transgene to avoid a possible immune reaction against the β-galactosidase protein. Animals were sacrificed 7, 14 and 21 days after the injection, and muscles analysed for the presence of immune infiltrate by immunofluorescence using antibodies against cell surface markers of murine leukocytes and macrophages.

No significant immune infiltrate was detected for up to 3 weeks after injection around the labelled cells, even though by this time all treated mice had developed antibodies against Ad5 proteins, which recognise adeno-infected but not control C3H fibroblasts

These experiments indicate that fibroblasts converted in vitro by exposure to an adeno-MyoD vector, and administered in vivo by intramuscular injection, do not elicit, and are therefore unlikely to be eliminated by, a cell-mediated cytotoxic immune response.

In contrast, direct injection of Adenoviral vector into regenerating muscle induced a strong immune infiltrate, as already reported (Yang, Y., et al., 1996).

### BIBLIOGRAPHY

Salvatori, G., G. Ferrari, A. Mezzogiorno, S. Serivdei, M. Coletta, P. Tonali, R. Giavazzi, G. Cossu, and F. Mavilio. 1993. Retroviral vector-mediated gene transfer into human primary myogenic cells leads to expression in muscle fibers in vivo. Human Gene Ther. 4:713-723.

Webster, C. e, H.M. Blau 1990. Accelerated age-related decline in replicative life span of Duchenne muscular dystrophy myoblasts: implication for cell and gene therapy. Somatic Cell Mol. Genet. 16:557-565.

Davis, R. L., H. Weintraub, and A.B. Lassar. 1987. Expression of a single transfected cDNA converts fibroblasts to myoblasts. Cell 51:987-1000.

Cossu, G., S. Tajbakhsh, and M. Buckingham. 1996. Myogenic specification in mammals. Trends Genet. 12:218-223.

Cossu, G. 1997.. Unorthodox myogenesis: possible developmental significance and implications for tissue histogenesis and regeneration. Histol. Histopathol. 12:755-760.

Breton, M., Z., Li, D. Paulin, J.A. Harris, F. Rieger, M. Pincon-Raymond, and L. Garcia. 1995. Myotube driven myogenic recruitment of cells during in vitro myogenesis. Develop. Dynam. 202:126-136.

Gibson, A.J., J. Karasinski, J. Relvas, J. Moss, T.G. Sherratt, P.N. Strong, and D.J. Watt. 1995. Dermal fibroblasts convert to a myogenic lineage in mdx mouse muscle. J. Cell Science 108:207-214.

Salvatori, G., L. Lattanzi, M. Coletta, S. Aguanno, E. Vivarelli, R. Kelly, G. Ferrari, J. A. Harris, F. Mavilio, M. Molinaro, and G. Cossu. 1995. Myogenic conversion of mammalian fibroblasts induced by differentiating muscle cells. J. Cell Science 108:2733-2739.

Murry, C. E., M. A. Kay, T. Bartosek, S. D. Hauschka, and S. M. Schwartz. 1996. Muscle differentiation during repair of myocardinal necrosis in rats via gene transfer with MyoD. J. Clin. Invest. 98:2209-2217.

Cusella - De Angelis, M.G., S. Molinari, A. Ledonne, M. Coletta, E. Vivarelli, M. Bouchè, M. Molinaro, S. Ferrari, and G. Cossu, 1994. Differential response of embryonic and fetal myoblasts to TGFβ: a possible regulatory mechanism of skeletal muscle histogenesis. Development 120:925-933.

Bader, D., T. Masaki, and D. A. Fischman. 1982. Immunochemical analysis of myosin heavy chain during avian myogenesis in vivo and in vitro. J. Cell Biol. 95:763-770.

Hasty, P., A. Bradley, J. H. Morris, D. G. Edmondson, J. M. Venuti, E. N. Olson, and W. H. Klein. 1993. Muscle deficiency and neonatal death in mice with a targeted mutation in the myogenin gene. Nature 375:787-790.

Edom, F., V. Mouly, J. P. Babert, M. Y. Fiszman, and G. S. Butler-Browne. 1994. Clones of human satellite cells can express in vitro both fast and slow myosin heavy chains. Develop. Biology 164:219-229.

Yasin, R., K. C. Van Beers, C. E. Nurse, S. Al-Ani, D. N. Landon, and E. J. Thompson. 1977. A quantitative technique for growing human adult skeletal muscle in culture starting from mononucleated cells. J. Neurol. Sci. 32:347-360.

Chomczynski, P., and N. Sacchi. 1987. Single-step method of RNA isolation by acid guanidinium thiocyanate-phenol-chlorophorm extraction. Anal. Biochem. 162:156-159.

Bober, E., G.E. Lyons, T. Braun, G. Cossu, M. Buckingham, and H.H. Arnold. 1991. The muscle regulatory gene Myf-6 has a biphasic pattern of expression during early mouse development. J. Cell Biol. 113:1255-1265.

Boulter, J., W. Luyten, K. Evans, P. Mason, M. Ballivet, D. Goldman, D. Stengelin, S. Martin, S. Heinemann, and J. Patric, 1985. Isolation of a clone coding for the α-subunit of a mouse acetylcholine receptor. J. Neurosci. 5:2545-2552.

Sambrook, J., E. F. Fritsch, and T. Maniatis. 1989. Molecular cloning: a laboratory manual. 2nd ed., Cold Spring Harbor Laboratory Press, New York.

Yang, Y., S. E. Haecker, Q. Su, and J. Wilson, 1996. Immunology of gene therapy with adenoviral vectors. Hum. Mol. Genet. 5:1703-1712.

Tajbakhrsh, S., E. Vivarelli, M. G. Cusella-De Angelis, D. Rocancourt, M. Buckingham, and G. Cossu 1994. A population of myogenic cells derived from the mouse neural tube. Neuron 13:813-821.

Lyons, G. E., S. Muhlebach, A. Moser, R., Masood, B. M. Paterson, M. Buckingham, and J. C. Perriard. 1991. Development regulation of creatin kinase gene expression by myogenic factors in embryonic mouse and chick skeletal muscle. Development 113:1017-1029.

## Claims

1. A method for the preparation of genetically-modified fibroblasts expressing a muscle lineage commitment gene, which comprises:
a) ex-vivo transduction of fibroblasts with a therapeutic gene or a gene capable of correcting a gene defect;
b) transient expression of the muscle lineage commitment gene in fibroblasts transduced as at point (a), through transformation of the cells with a high-efficiency DNA transfer method, wherein the muscle lineage commitment gene is under the control of a strong promoter.

2. A method according to claim 1, wherein the therapeutical gene is the dystrophin gene.

3. A method according to claim 1, wherein the high-efficiency DNA transfer method is a viral vector.

4. A method according to claim 3, wherein said viral vector is selected from baculovirus, adeno-related viruses, adeno-virus.

5. A method according to claim 3, wherein said vector is an adenovirus.

6. A method according to claim 1, wherein the muscle lineage commitment gene is selected from MyoD, Myf-5, MRF4 and myogenin.

7. A method according to claim 6, wherein said gene is MyoD.

8. A method according to claim 1, wherein the muscle lineage commitment gene is under the control of a viral promoter.

9. Genetically-modified fibroblasts obtainable by the method of claims 1-8.

10. Fibroblasts according to claim 9, wherein the muscle lineage commitment gene is MyoD.

## Patentansprüche

1. Verfahren zur Herstellung von genetisch modifizierten Fibrobalsten, die ein Muskelzelldifferenzierungsgen exprimieren, welches umfasst:
a) ex-vivo-Transduktion von Fibroblasten mit einem therapeutischen Gen oder einem Gen, das zur Korrektur eines Gendefekts fähig ist;
b) transiente Expression des Muskelzelldifferenzierungsgens in wie im Punkt a) transduzierten Fibroblasten durch Transformation der Zellen mit einer hocheffizienten DNA-Übertragungsmethode, wobei das Muskelzelldifferenzierungsgen unter der Kontrolle eines starken Promoters steht.

2. Verfahren nach Anspruch 1, wobei das therapeutische Gen das Dystrophin-Gen ist.

3. Verfahren nach Anspruch 1, wobei die hocheffiziente DNA-Übertragungsmethode ein viraler Vektor ist.

4. Verfahren nach Anspruch 3, wobei der virale Vektor ausgewählt wird aus Bakulovirus, Adeno-verwandten Viren, Adenovirus.

5. Verfahren nach Anspruch 3, wobei der Vektor ein Adenovirus ist.

6. Verfahren nach Anspruch 1, wobei das Muskelzelldifferenzierungsgen ausgewählt wird aus MyoD, Myf-5, MRF4 und Myogenin.

7. Verfahren nach Anspruch 6, wobei das Gen MyoD ist.

8. Verfahren nach Anspruch 1, wobei das Muskelzelldifferenzierungsgen unter der Kontrolle eines viralen Promoters steht.

9. Genetisch modifizierte Fibroblasten, erhältlich durch das Verfahren nach den Ansprüche 1 bis 8.

10. Fibroblasten nach Anspruch 9, wobei das Muskelzelldifferenzierungsgen MyoD ist.

## Revendications

1. Procédé pour la préparation de fibroblastes modifiés génétiquement exprimant un gène d'obligation de lignage musculaire, qui comprend :
a) la transduction *ex-vivo* de fibroblastes avec un gène thérapeutique ou un gène apte à corriger un défaut génétique ;
b) l'expression transitoire du gène d'obligation de lignage musculaire dans les fibroblastes transduits comme au point (a), par transformation des cellules avec un procédé de transfert d'ADN de haute efficacité, dans lequel le gène d'obligation de lignage musculaire est sous le contrôle d'un promoteur fort.

2. Procédé selon la revendication 1, dans lequel le gène thérapeutique est le gène de la dystrophine.

3. Procédé selon la revendication 1, dans lequel le procédé de transfert d'ADN de haute efficacité est un vecteur viral.

4. Procédé selon la revendication 3, dans lequel ledit vecteur viral est choisi parmi un baculovirus, les virus adéno-apparentés, un adénovirus.

5. Procédé selon la revendication 3, dans lequel ledit vecteur est un adénovirus.

6. Procédé selon la revendication 1, dans lequel le gène d'obligation de lignage musculaire est choisi parmi MyoD, Myf-5, MRF4 et la myogénine.

7. Procédé selon la revendication 6, dans lequel ledit gène est MyoD.

8. Procédé selon la revendication 1, dans lequel le gène d'obligation de lignage musculaire est sous le contrôle d'un promoteur viral.

9. Fibroblastes modifiés génétiquement pouvant être obtenus par le procédé selon les revendications 1 à 8.

10. Fibroblastes selon la revendication 9, dans lequel le gène d'obligation de lignage musculaire est MyoD.
